Europäisches Patentamt

European Patent Office

Office européen des brevets

(19)

(11) Veröffentlichungsnummer: **0 234 186 B1**

(12) # EUROPÄISCHE PATENTSCHRIFT

(45) Veröffentlichungstag der Patentschrift: 06.11.91

(51) Int. Cl.⁵: **A61K 31/195**, A61K 37/02

(21) Anmeldenummer: **87100072.5**

(22) Anmeldetag: **06.01.87**

Die Akte enthält technische Angaben, die nach dem Eingang der Anmeldung eingereicht wurden und die nicht in dieser Patentschrift enthalten sind.

(54) **Verwendung von Oligopeptiden zur Behandlung von cerebralen Störungen.**

(30) Priorität: **18.01.86 DE 3601398**

(43) Veröffentlichungstag der Anmeldung:
**02.09.87 Patentblatt 87/36**

(45) Bekanntmachung des Hinweises auf die
Patenterteilung:
**06.11.91 Patentblatt 91/45**

(84) Benannte Vertragsstaaten:
**AT BE CH DE ES FR GB GR IT LI LU NL SE**

(56) Entgegenhaltungen:
**EP-A- 0 221 019**

**CHEMICAL ABSTRACTS, Band 102, Nr. 24, 17. Juni 1985, Seite 625, Spalte 2, Abstract Nr. 221172j, Dep. Chem. Peking University, Rep.Pop.CHINA; YE, YUNHUA et al.: "Application of a new active amide method in the synthesis of delta sleep-inducing peptide (DSIP)", & BEIJING DAXUE XUEBAO 1984, (4), 63-69**

(73) Patentinhaber: **Fresenius AG**
**Gluckensteinweg 5**
**W-6380 Bad Homburg v.d.H.(DE)**

(72) Erfinder: **Sommermeyer, Klaus, Dr. rer. nat.**
**Kapersburgstrasse 6b**
**W-6365 Rosbach v.d.H.(DE)**
Erfinder: **Weidler, Burghard, Dr. med.**
**Friedrich-Ebert-Strasse 6**
**W-6365 Rosbach 1 v.d.H.(DE)**

(74) Vertreter: **Dr. Fuchs, Dr. Luderschmidt Dipl.-Phys. Seids, Dr. Mehler Patentanwälte**
**Abraham-Lincoln-Strasse 7**
**W-6200 Wiesbaden(DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

CHEMICAL ABSTRACTS, Band 99, Nr. 13, 26.09.1983, Seite 627, Spalte 2, Abstract Nr. 105692z, Dep. Chem. Beijing University Rep. Pop. China; JI, AIXUEet al.:"Synthesis of delta sleep-inducing peptide (DSIP) and its physiological activity", & SCI. SIN., SER. B (ENG1. ED.) 26 (2), 174-185

CHEMICAL ABSTRACTS, Band 93, Nr. 4, 28 Juli 1980, Seite 990, Spalte 1, Abstract Nr. 47190x, Dep. Chem. Peking University, Rep. Pop. China; CHI, AI-HSEEH et al.: "Synthesis of delta sleep-inducing peptide and its 7-glycine analog"

CHEMICAL ABSTRACTS, Band 87, Nr. 13, 26 September 1977, Seite 109, Spalte 1, Abstract Nr. 96213s, Tufts University, Boston, Mass., US; HARTMANN et al.: "The insomnia of sleeping in a strange place: effects of L-trytophane"

## Beschreibung

Gegenstand der vorliegenden Erfindung ist die Verwendung von Oligopeptiden, wie sie in Patentanspruch 1 gekennzeichnet sind, zur Herstellung eines Arzneimittels zur Behandlung von cerebralen Störungen, insbesondere von Schlaflosigkeit und Depressionen.

Schlaflosigkeit und Depressionen sind bekannte Probleme, denen täglich zahlreiche Personen gegenüberstehen. Zwar existiert eine Fülle von zuverlässig wirkenden Arzneimitteln, jedoch können alle diese wegen ihrer unterschiedlichen Nebeneffekte nicht bedenkenlos eingesetzt werden. Bei der oft notwendigen Dauertherapie mahnen Gewöhnungseffekte, vor allem bei den Benzodiazepinen, zur Vorsicht.

Aber auch Barbiturate und andere gängige Schlafmittel können zur Abhängigkeit führen, stellen ein Suizidrisiko dar und haben eine Vielzahl von Nebenwirkungen.

Aus Chemical Abstracts Vol. 93 (1980), Seite 990, No. 47190x, Vol. 99 (1983), Seite 627, No. 105692z, und Vol. 102 (1985), No. 221172j ist das Nonapeptid "sleep inducing peptide", Verfahren zu dessen Synthese und dessen physiologische Wirksamkeit bekannt. Das Nonapeptid, ursprünglich als humoraler Schlaffaktor aus Kaninchenblut isoliert, wird, da es die δ -Welle im EEG erhöhte, als "delta sleep-inducing peptide (DSIP)" bezeichnet. Seine physiologische Funktion ist von der gängiger Schlafmittel verschieden.

Die in jüngster Zeit über die Physiologie des Schlafes gewonnenen grundlegenden Erkenntnisse bestätigen die Komplexizität des Schlafablaufes und zeigen auch auf, wie komplex die physiologischen und biochemischen Mechanismen, die den Schlaf auslösen und den ordnungsgemäßen Ablauf der einzelnen Schlafphasen garantieren, sind.

An solchen Mechanismen sind verschiedene Hirnareale mit unterschiedlichen Überträgerstoffen, sog. Neurotransmittern, beteiligt, die meist aus Aminosäuren synthetisiert werden.

Als solche Neurotransmitter bekannt sind Noradrenalin und Dopamin (synthetisiert aus Tyrosin) und Serotonin, das aus Tryptophan synthetisiert wird. Untersuchungen haben gezeigt, daß bei Mangelzuständen bestimmter Neurotransmitter oder deren Vorstufe die Reizleitung beeinträchtigt sein kann, was dann wiederum oft psychische Erkrankungen, wie z. B. Schlafstörungen und Depressionen zur Folge hat. Der Schlaf wird durch serotonerge Neuronen im sog. Raphe-System im Grenzbereich von Mittel- und Rautenhirn ausgelöst. Wird die Biosynthese von Serotonin aus Tryptophan im Gehirn gehemmt, dann tritt als Folge davon Schlaflosigkeit auf. Serotoninmangel läßt sich nicht durch direkte Substitution beheben, da Serotonin, unabhängig von unerwünschten Nebenwirkungen, die sog. Blut-Hirnschranke selbst nicht zu überwinden vermag. Es muß daher in den serotonergen Neuronen aus der essentiellen Aminosäure L-Tryptophan gebildet werden. Diese Synthese geschieht in 2 Schritten, wobei zunächst durch Hydroxylierung mit Tryptophan-Hydroxylase 5-Hydroxytryptophan und aus diesem durch Decarboxylierung durch aromatische L-Aminosäuren-Decarboxylase 5-Hydroxytryptamin, d.h. Serotonin gebildet wird.

Ein Mangel an L-Tryptophan erzeugt so unter anderem Schlafstörungen und Depressionen. Tryptophanmangel und seine Auswirkungen wurden daher erfolgreich durch Verabreichung von L-Tryptophan, das in freier Form die Blut- Hirnschranke durchdringen kann, behandelt. Eine solche Verabreichung besitzt gegenüber den klassischen Antidepressiva, die die Inaktivierung der zentra-len Überträgerstoffe, wie des Serotonins, hemmen, und den herkömmlichen Hypnotika, die unspezifisch die aktivierende Formatio reticoluris dämpfen, den Vorteil einer kausalen und nebenwirkungsfreien Therapie und daß sie nicht zur Abhängigkeit oder Sucht führt und kein Suizidrisiko bedeutet und außerdem die Möglichkeit bietet, bei schweren Störungen, die die Behandlung mit eigentlichen Psychopharmaka erfordern, deren Dosis durch Verabreichung von Tryptophan zu reduzieren (vergl. "Der Deutsche Apotheker", 35. Jahrgang, Heft 4, 1983, Seiten 1 - 7).

Ein gewisser Nachteil dieser Behandlung mit L-Tryptophan, das nur in freier Form die Blut- Hirnschranke zu durchdringen vermag, besteht in der relativ eingeschränkten Resorption bzw. Resorbierbarkeit des L-Tryptophan aus dem Gastrointestinal-Trakt und des mangelhaften Transportes des L-Tryptophans durch körpereigene Membranen, so daß L-Tryptophan in hoher Dosis und hoher Einnahmefrequenz verabreicht werden muß. Zur Behandlung cerebraler Störungen ist es wesentlich, daß der Serotoningehalt im Hirn hoch und im Blut niedrig gehalten wird, da andernfalls ein hoher Serotoningehalt im Blut zu peripheren Störungen führen würde.

Es wurde auch bereits versucht, anstelle von Serotonin oder L-Tryptophan den Serotonin-Precursor 5-Hydroxy-L-tryptophan zu verwenden. Diese Verwendung birgt die Gefahr in sich, daß 5-Hydroxy-L-tryptophan bereits im Blut durch Decarboxylase in Serotonin umgewandelt wird und so die Konzentration von Serotonin im Blut unerwünscht erhöht wird. Dabei werden vor allem gastrointestinale Nebenwirkungen, wie Übelkeit und Diarrhoe beobachtet. Um das zu verhindern, mußte 5-Hydroxy-L-tryptophan mit einem Decarboxylase-Hemmer, wie Benserazide (INN) - d.h. N-(DL-Seryl)-N'-(2,3,4-trihydroxy-benzyl)-hydrazin oder Carbidopa (INN), d.h. (-)-L-α-Hydrazino-3,4-di-hydroxy-α-methyl-hydrozimtsäure gemeinsam verabreicht werden. Diese Decarboxylase-Hemmer haben jedoch den Nachteil, daß sie unerwünschte und teilweise erhebliche Nebenwirkungen erzeugen.

Es besteht daher weiterhin ein Bedarf nach geeigneten Mitteln für die Therapie von cerebralen Störungen, insbesondere Schlaflosigkeit und Depressionen.

Der vorliegenden Erfindung lag daher die Aufgabe zugrunde, ein neues Mittel zur Herstellung eines Arzneimittels zur Behandlung von cerebralen Störungen, insbesondere Schlaflosigkeit und Depressionen zu verwenden, das die Nachteile der bekannten Mittel nicht aufweist, die Verwendung von Decarboxylase-Hemmern überflüssig macht, das nicht toxisch ist, im wesentlichen keine Nebenwirkungen besitzt, nicht zu Abhängigkeit oder Sucht führt, kein Suizidrisiko bedeutet und vom Körper gut resorbiert wird, d.h. die Verwendung geringer Mengen ermöglicht und einen besseren Transport durch körpereigene Membranen zeigt.

Erfindungsgemäß wurde überraschend gefunden, daß diese Aufgabe durch Verwendung eines Oligopeptides oder mehrerer Oligopeptide der Formel 1, gegebenenfalls in Kombination mit L-Tryptophan, gelöst werden kann.

Die erfindungsgemäß verwendeten Oligopeptide besitzen die allgemeine Formel

HX-(Y)$_n$-OH    I,

worin H ein Wasserstoffatom, X einen von Tryptophan, 5-Hydroxy-L-tryptophan oder einer der L-Aminosäuren: Glycin, Alanin, Serin, Threonin, Cystein, Methionin, Asparagin, Asparaginsäure, Glutamin, Glutaminsäure, Histidin, Lysin und Prolin abgeleiteten Rest, Y einen von L-Tryptophan, 5-Hydroxy-L-tryptophan oder einer der vorstehend genannten anderen Aminosäuren abgeleiteten Rest und n 1 oder 2 bedeuten, wobei, wenn n 2 ist, die Reste Y von gleichen oder verschiedenen Aminosäuren abgeleitet sein können, und wobei wenigstens einer der Reste X oder Y den von L-Tryptophan oder 5-Hydroxy-L-tryptophan abgeleiteten Rest bedeutet. Vorzugsweise bedeutet wenigstens einer der Reste X oder Y den von L-Tryptophan abgeleiteten Rest.

Vorzugsweise verwendet werden erfindungsgemäß solche Oligopeptide der vorstehend genannten allgemeinen Formel I, worin H, X und Y die vorstehend angegebene Bedeutung besitzen und n beispielsweise 1 bedeutet.

Als andere Aminosäure, außer L-Tryptophan und 5-Hydroxy-L-tryptophan, von der die Reste X und/oder Y abgeleitet sind und die in den erfindungsgemäß verwendeten Oligopeptiden verknüpft sein kann, wird insbesondere Glycin verwendet.

Erfindungsgemäß können in den erfindungsgemäß verwendeten Oligopeptiden X und Y von gleichen oder verschiedenen Aminosäuren abgeleitet sein unter der Maßgabe, daß mindestens eine der verknüpften Aminosäuren L-Tryptophan oder 5-Hydroxy-L-tryptophan, vorzugsweise L-Tryptophan ist. So können z.B. auch alle Reste von L-Tryptophan und/oder 5-Hydroxy-L-tryptophan abgeleitet sein.

Weiterhin können nicht nur die reinen Oligopeptide, sondern auch Gemische verschiedener Oligopeptide der vorstehend genannten Formel I sowie geeignete pharmazeutisch verträgliche Salze der Oligopeptide verwendet werden. Geeignete pharmazeutisch verträgliche Salze sind beispielsweise die entsprechenden Acetate.

Beispiele für geeignete Oligopeptide sind:

L-Tryptophyl-L-tryptophan, L-Alanyl-L-tryptophan, L-Tryptophyl-L-alanin, L-Glycyl-L-tryptophan, L-Tryptophyl-L-glycin, 5-Hydroxy-L-tryptophyl-L-glycin, 5-Hydroxy-L-tryptophyl-L-alanin, L-Glycyl-5-hydroxy-L-tryptophan, L-Alanyl-5-hydroxy-L-tryptophan, L-Tryptophyl-L-tryptophyl-L-tryptophan, L-Alanyl-L-glycyl-L-tryptophan, L-Tryptophyl-L-alanyl-L-glycin, L-Alanyl-L-tryptophyl-L-glycin, L-Glycyl-L-tryptophyl-L-alanin.

Beispiele für bevorzugte Verbindungen sind:

L-Tryptophyl-L-tryptophan, L-Alanyl-L-tryptophan, L-Tryptophyl-L-alanin, L-Glycyl-L-tryptophan, L-Tryptophyl-L-glycin, 5-Hydroxy-L-tryptophyl-L-glycin, 5-Hydroxy-L-tryptophyl-L-alanin, L-Glycyl-5-hydroxy-L-tryptophan, L-Alanyl-5-hydroxy-L-tryptophan, L-Tryptophyl-L-tryptophyl-L-tryptophan, L-Alanyl-L-glycyl-L-tryptophan, L-Tryptophyl-L-alanyl-L-glycin, L-Alanyl-L-tryptophyl-L-glycin, L-Glycyl-L-tryptophyl-L-alanin.

Besonders bevorzugt sind erfindungsgemäß L-Tryptophyl-L-tryptophan, L-Alanyl-L-tryptophan, L-Tryptophyl-L-alanin, L-Glycyl-L-tryptophan, L-Tryptophyl-L-glycin. Insbesondere bevorzugt ist L-Glycyl-L-tryptophan.

Die erfindungsgemäß verwendeten Oligopeptide können in bekannter Weise hergestellt werden, wobei auch sog. gentechnische Methoden eingesetzt werden können. Anstelle der reinen Oligopeptide oder deren Gemische können erfindungsgemäß auch Hydrolysate, die diese Oligopeptide enthalten, verwendet werden.

Auch Gemische von Tryptophan mit den vorstehend genannten Oligopeptiden können vorteilhaft eingesetzt werden. Ein Beispiel für ein bevorzugtes Gemisch ist ein Gemisch aus L-Tryptophan und L-Glycyl-L-tryptophan.

Werden die Oligopeptide in Kombination mit L-Tryptophan verwendet, so können sie im allgemeinen im Gewichtsverhältnis von 1 : 10 bis 10 : 1, vorzugsweise im Gewichtsverhältnis von 1 : 5 bis 5 : 1 und insbesondere im Gewichtsverhältnis von 1 : 2 bis 2 : 1, beispielsweise im Gewichtsverhältnis von 1 : 1, eingesetzt werden. Bevorzugt ist ein Gemisch aus L-Tryptophan und L-Glycyl-L-tryptophan im Verhältnis 1 : 1.

Die erfindungsgemäß verwendeten Oligopeptide zeigen den bekannten, zur Behandlung von cerebralen Störungen, insbesondere Schlafstörungen und Depressionen eingesetzten Mitteln gegenüber erhebliche Vorteile. Sie sind nicht toxisch, besitzen keine Nebenwirkungen, führen nicht zu Abhängigkeit oder Sucht, bedingen kein Suizidrisiko, werden ausgezeichnet vom Körper resorbiert, zeigen erheblich besseres pharmakokinetisches Verhalten und besseren Transport durch körpereigene Membranen als Tryptophan selbst und machen die Verwendung von Decarboxylase-Hemmern überflüssig. Sie können darüber hinaus in geringeren Mengen und niedrigeren Konzentrationen verwendet werden. Überraschenderweise wurde ferner gefunden, daß Gemische aus L-Tryptophan mit den genannten Oligopeptiden bei der Behandlung von cerebralen Störungen, insbesondere Schlafstörungen und Depressionen, zu ausgezeichneten Ergebnissen führen.

Die erfindungsgemäß erzielbaren vorteilhaften Ergebnisse wurden durch Untersuchungen bestätigt.

Die erfindungsgemäß zu verwendenden Oligopeptide bzw. Kombinationen der Oligopeptide mit L-Tryptophan können so formuliert werden, daß sie sich für die orale Verabreichung eignen. Für derartige Zwecke werden sie mit bekannten Verdünnungsmitteln gemischt und anschließend zu bekannten Verabreichungsformen, wie Tabletten, Kapseln, Suspensionen, Emulsionen, dispergierbaren Pulvern, Sirupen und Elixieren verarbeitet.

Oligopeptide oder Kombinationen derselben mit L-Tryptophan, die relativ wasserunlöslich sind, können als Suspension in hochprozentiger wäßriger Sorbit- oder Glycerinlösung mit einem Zusatz von Suspensionshilfsmitteln sowie entsprechenden Aromen verarbeitet werden.

Flüssige und feste Formulierungen können in Kapseln für die orale Verabreichung gefüllt werden. Aktive Wirkstoffe können in Ölen pflanzlichen oder tierischen Ursprungs, beispielsweise Sonnenblumenöl, Sojaöl, Maisöl oder Lebertran suspendiert oder gelöst werden und sie können zusätzliche Bestandteile, beispielsweise Antioxidantien, enthalten.

Feste Formulierungen, die zum Abfüllen in Kapseln geeignet sind, können die Wirkstoffe in Vermischung mit üblichen festen Trägersubstanzen enthalten.

Tabletten können in an sich bekannter Weise hergestellt werden, wobei sie gegebenenfalls überzogen hergestellt werden können. Als inerte Verdünnungsmittel oder Träger können beispielsweise Magnesiumcarbonat oder Lactose in Verbindung mit üblichen Sprengmitteln, beispielsweise, Maisstärke oder Alginsäuren, und/oder Gleitmitteln, beispielsweise Magnesiumstearat, in Frage kommen.

Die nachstehenden Beispiele dienen der weiteren Erläuterung der vorliegenden Erfindung:

Beispiel 1:

1.500 Teile L-Alanyl-L-tryptophan (Fp. 293-294° C, $[\alpha]_D^{20}$ + 15,5 ± 0,5°)
60 Teile Maisstärke
100 Teile Polyvinylpyrrolidon
30 Teile Polyvinylpolypyrrolidon und
2 Teile hochdisperses Siliziumdioxid
wurden zu einem Granulat verarbeitet. Dieses Granulat wurde durch ein Sieb der lichten Maschenweite von 1,5 mm passiert. Anschließend wurden weitere
2 Teile hochdisperses Siliziumdioxid
30 Teile Magnesiumstearat
30 Teile Talkum
30 Teile Polyvinylpolypyrrolidon sowie
250 Teile Maisstärke
zugesetzt, und das erhaltene Gemisch anschließend in Tabletten gepreßt, die sich für die orale Verabreichung eigneten.

Beispiel 2:

1.500 Teile L-Alanyl-L-tryptophan, 100 Teile Maisstärke und 10 Teile Alginsäure wurden mit einer wäßrigen 15 %igen Maisstärkepaste gemischt und verarbeitet und anschließend granuliert. Das Granulat wurde durch ein Sieb mit einer lichten Maschenweite von 1,5 mm passiert und anschließend bei ca. 60° C getrocknet. Das Granulat wurde erneut durch ein Sieb mit der lichten Maschenweite von 1,5 mm passiert und mit 10 Teilen Magnesiumstearat versetzt. Das so erhaltene Gemisch wurde anschließend zu Tabletten gepreßt, die für die orale Verabreichung geeignet waren.

Beispiel 3:

600 Teile L-Glycyl-L-tryptophan
$F_p$ 235° C, $[\alpha]_D^{24}$ + 34° (5N HCl) wurden in einem Gemisch aus 300 Teilen Wasser und 700 Teilen Sorbit suspendiert. Nach Zusatz eines geeigneten Geschmacksstoffes und Farbstoffes erhielt man eine für die orale Verabreichung geeignete Suspension.

Beispiel 4:

50 Teile L-Alanyl-L-tryptophan wurden in 941 Teilen Wasser für Injektionszwecke gelöst. Die Lösung wurde durch Zusatz von 3 g Natriumchlorid isotonisiert und nach pH-Wert-Einstellung mit Natronlauge oder Salzsäure auf pH 5,5 - 6,0 durch 0,2 μm Membranfilter keim-und partikelfrei filtriert und in Infusionsflaschen abgefüllt. Nach Verschließen der Behältnisse wurden diese in einem Dampfautoklaven sterilisiert.

In analoger Weise lassen sich auch L-Glycyl-L-tryptophan oder andere geeignete 01igopeptide enthaltende Infusionslösungen herstellen.

Beispiel 5:

Unter Verwendung von 1.500 Teilen eines Gemisches aus L-Glycyl-L-tryptophan und L-Tryptophan (Verhältnis ist 1 : 1) anstelle von 1.500 Teilen L-Alanyl-L-tryptophan wurde unter Verwendung der im Beispiel 1 angegebenen weiteren Bestandteile gemäß der Verfahrensweise von Beispiel 1 ein Präparat in Tablettenform, das sich für die orale Verabreichung eignet, hergestellt.

Beispiel 6:

1.500 Teile eines Gemisches aus L-Glycyl-L-tryptophan und L-Tryptophan (Verhältnis 1 : 1), 100 Teile Maisstärke und 10 Teile Alginsäure wurden mit einer 15 %igen wäßrigen Maisstärkepaste gemischt und verarbeitet und anschließend granuliert. Das Granulat wurde durch ein Sieb mit der lichten Maschenweite von 1,5 mm passiert und anschließend bei ca. 60° C getrocknet. Das Granulat wurde erneut durch ein Sieb mit der lichten Maschenweite von 1,5 mm passiert und mit 10 Teilen Magnesiumstearat versetzt. Das so erhaltene Gemisch wurde anschließend zu Tabletten verpreßt, die für die orale Verabreichung geeignet waren.

Beispiel 7:

60 Teile eines Gemisches aus L-Alanyl-L-tryptophan und L-Tryptophan (Verhältnis ist 5 : 1) wurden in 931 Teilen Wasser für Injektionszwecke gelöst. Die Lösung wurde durch Zusatz von 1,5 g Natriumchlorid isotonisiert und nach pH-Wert-Einstellung mit Natronlauge oder Salzsäure auf 5,5 - 6,0 durch 0,2 μm Membranfilter keim- und partikelfrei filtriert und in Infusionsflaschen abgefüllt. Nach Verschließen der Behältnisse wurden diese in einem Dampfautoklaven sterilisiert.

In analoger Weise lassen sich auch Infusionslösungen herstellen, die L-Glycyl-L-tryptophan oder andere geeignete Oligopeptide im Gemisch mit L-Tryptophan enthalten.

Beispiel 8:

600 Teile eines Gemisches aus L-Glycyl-L-tryptophan und L-Tryptophan (Verhältnis ist 1 : 1) wurden in einem Gemisch aus 300 Teilen Wasser und 700 Teilen Sorbit suspendiert. Nach Zusatz eines geeigneten Geschmacksstoffes wurde eine für die orale Verabreichung geeignete Suspension erhalten.

Untersuchung der Resorption von L-Glycyl-L-tryptophan im Vergleich zu L-Tryptophan bei Ratten:

Als Versuchstiere wurden Sprague-Dawley-Ratten bei üblicher Versuchstierhaltung verwendet. Den Ratten wurden nach 18stündigem Fasten die zu untersuchenden Substanzen per Schlundsonde appliziert. Im Versuch wurden die beiden Substanzen gegeneinander getestet. Die Dosierung richtete sich nach dem Körpergewicht der Versuchsratten. Die Ratten erhielten 13,3 μmol L-Tryptophan bzw. 6,7 μmol L-Glycyl-L-tryptophan in 0,4 ml Wasser/100 g Körpergewicht. Zur Untersuchung der Resorption der zu untersuchenden Substanzen wurde den Ratten aus der Schwanzspitze jeweils 50 μl Blut (in heparinisierten Hämatokritkapillaren) entnommen. Die Blutentnahmen erfolgten unmittelbar vor Applikation und 5, 10, 20, 30, 45, 60, 90, 120, 180 und 240 Minuten nach der Applikation.

Die Gewinnung des Rattenbluts in Kapillaren ermöglichte gleichzeitig die Bestimmung des Hamatokrits und somit die mögliche Veränderung des Blutvolumens durch die mehrfache Blutentnahme.

Zur Blutuntersuchung wurde das Blutplasma verwendet, wobei 20 μl mit 200 μl 0,4 N Perchlorsäure versetzt wurden und anschließend zentrifugiert wurde. Der erhaltene Überstand wurde analysiert. Als Nachweisverfahren diente die Auftrennung über eine C18 SIL-X10 Säule (25 × 0,4 + 5 cm × 0,4) mit nachfolgender fluorometrischer Detektion bei 276/350 nm. Als Elutionspuffer wurde 0,05 M Natriumacetat/0,05 M Citronensäure/17 % Methanol, pH 4,2, verwendet.

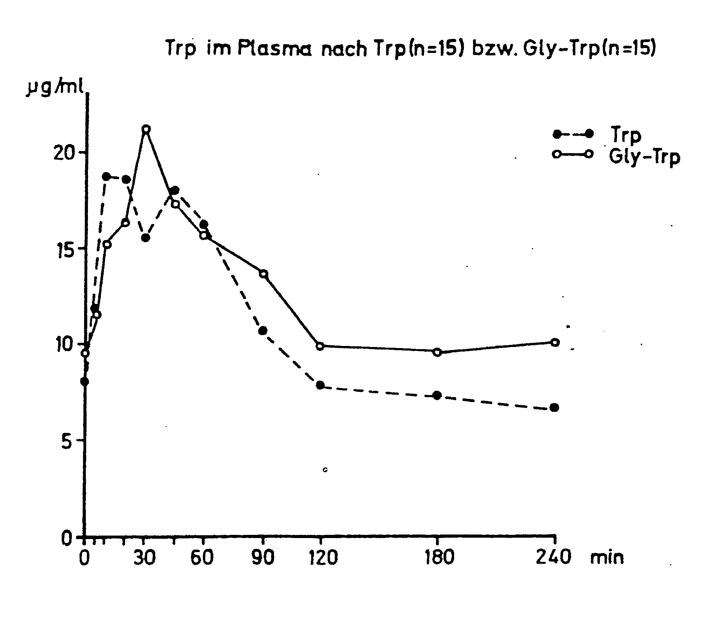

Die bei der Untersuchung erhaltenen Ergebnisse sind aus der beiliegenden Figur ersichtlich. In dieser Figur bedeutet n die Anzahl der Ratten, der Ausdruck Trp L-Tryptophan und Gly-Trp L-Glycyl-L-tryptophan.

**Patentansprüche**

1. Verwendung von Oligopeptiden der allgemeinen Formel I

$$HX\text{-}(Y)_n\text{-}OH \qquad I,$$

worin H ein Wasserstoffatom, X einen von Tryptophan, 5-Hydroxy-L-tryptophan oder einer der L-Aminosäuren: Glycin, Alanin, Serin, Threonin, Cystein, Methionin, Asparagin, Asparaginsäure, Glutamin, Glutaminsäure, Histidin, Lysin und Prolin abgeleiteten Rest, Y einen von L-Tryptophan, 5-Hydroxy-L-tryptophan oder einer der vorstehend genannten anderen Aminosäuren abgeleiteten Rest und n 1 oder 2 bedeuten, wobei, wenn n 2 ist, die Reste Y von gleichen oder verschiedenen Aminosäuren abgeleitet sein können, und wobei wenigstens einer der Reste X oder Y den von L-Tryptophan oder 5-Hydroxy-L-tryptophan abgeleiteten Rest bedeutet, zur Herstellung eines Arzneimittels zur Behandlung von cerebralen Störungen, insbesondere Schlaflosigkeit und Depression.

2. Verwendung nach Anspruch 1, dadurch gekennzeichnet, daß solche Oligopeptide der allgemeinen Formel I, worin n = 1 bedeutet und H, X und Y die in Anspruch 1 angegebene Bedeutung besitzen, verwendet werden.

3. Verwendung nach Anspruch 2, dadurch gekennzeichnet, daß als Oligopeptid L-Glycyl-L-tryptophan verwendet wird.

4. Verwendung nach Anspruch 1 oder 2, dadurch gekennzeichnet, daß man das Oligopeptid im Gemisch mit L-Tryptophan verwendet.

5. Verwendung nach Anspruch 1 - 4, dadurch gekennzeichnet, daß die Oligopeptide zusammen mit üblichen Hilfs- und Trägerstoffen in den üblichen Darreichungsformen verwendet werden.

## Claims

1. Use of oligopeptides of the general formula I

   $$HX - (Y)_n - OH \qquad I$$

   wherein H signifies a hydrogen atom, X signifies a residue derived from tryptophan, 5-hydroxy-L-tryptophan or from one of the L-amino acids: Gly, Ala, Ser, Thr, Cys, Mex, Asn, Asp, Gln, Glu, His, Lys and Pro, Y signifies a residue derived from tryptophan, 5-hydroxy-L-tryptophan or one of the previously mentioned other amino acids and n denotes 1 or 2, and wherein, in case that n denotes 2, the residues Y may derive from the same or different amino acids, and at least one of the residues X or Y signifies the residue derived from L-tryptophan or 5-hydroxy-L-tryptophan, in order to prepare a remedy for the treatment of cerebral disorders, especially of insomnia and of depression.

2. Use according to claim 1, characterized in that such oligopeptides of the general formula I are used in which n denotes 1 and H, X and Y have the meaning given in claim 1.

3. Use according to claim 2, characterized in that Gly-Trp is used as oligopeptide.

4. Use according to claim 1 or 2, characterized in that the oligopeptide mixed with Trp is used.

5. Use according to claims 1 to 4, characterized in that the oligopeptides are used in the usual modes of administration together with conventional auxiliary and carrier materials.

## Revendications

1. Utilisation d'oligopeptides de la formule générale I :

   $$HX-(Y)_n-OH \qquad I$$

   où H représente un atome d'hydrogène, X représente un reste dérivé du tryptophane, du 5-hydroxy-L-tryptophane ou de l'un des L-acides aminés : glycine, alanine, sérine, thréonine, cystéine, méthionine, asparagine, acide aspargique, glutamine, acide glutamique, histidine, lysine et proline, Y représente un reste dérivé du L-tryptophane, du 5-hydroxy-L-tryptophane ou de l'un des autres acides aminés mentionnés ci-dessus, et n représente 1 ou 2, étant entendu que lorsque n est 2, les restes Y peuvent dériver d'acides aminés identiques ou différents et qu'au moins l'un des restes X ou Y représente le reste dérivé du L-tryptophane ou du 5-hydroxy-L-tryptophane, pour la préparation d'un médicament propre au traitement de désordres cérébraux, en particulier de l'insomnie et des dépressions.

2. Utilisation suivant la revendication 1, caractérisée en ce que des oligopeptides de formule générale I, où n représente 1 et H, X et Y ont les significations indiquées dans les revendications 1, sont mis en oeuvre.

3. Utilisation suivant la revendication 2, caractérisée en ce que le L-glycyl-L-tryptophane est mis en oeuvre comme oligopeptide.

4. Utilisation suivant la revendication 1 ou 2, caractérisée en ce l'oligopeptide est mis en oeuvre en mélange avec du L-tryptophane.

5. Utilisation suivant l'une quelconque des revendications 1 à 4, caractérisée en ce que les oligopeptides sont mis en oeuvre conjointement avec des substances auxiliaires et des excipients habituels dans les formes d'admi-

nistration classiques.

Trp im Plasma nach Trp(n=15) bzw. Gly-Trp(n=15)